(19) Europäisches Patentamt
European Patent Office
Office européen des brevets

(11) Publication number: **0 630 652 A2**

(12) # EUROPEAN PATENT APPLICATION

(21) Application number: **94202430.8**

(51) Int. Cl.$^5$: **A61K 49/00**, A61K 49/04

(22) Date of filing: **29.10.92**

This application was filed on 24 - 08 - 1994 as a divisional application to the application mentioned under INID code 60.

(30) Priority: **30.10.91 GB 9122984**

(43) Date of publication of application:
**28.12.94 Bulletin 94/52**

(60) Publication number of the earlier application in accordance with Art.76 EPC: **0 610 398**

(84) Designated Contracting States:
**AT BE CH DE DK ES FR GB GR IE IT LI LU MC NL SE**

(71) Applicant: **NYCOMED SALUTAR, INC.**
**428 Oakmead Parkway**
**Sunnyvale,**
**California 94086 (US)**

(72) Inventor: **Fellmann, Jere Douglas**
**1474 Lexington Way**
**Livermore, CA 94550 (US)**

(74) Representative: **Matthews, Derek Peter**
**Frank B. Dehn & Co.**
**Imperial House**
**15-19 Kingsway**
**London WC2B 6UZ (GB)**

(54) **Contrast media.**

(57) There are provided improved diagnostic metal containing molecular sieves for use as contrast agents in diagnostic imaging techniques such as X-ray, scintigraphy and, especially, MRI. For MRI the diagnostic metals are paramagnetic metal ions. Reduced risk of toxic metal release in vivo, especially into the GI tract, is achieved by surface modification of the molecular sieve.

EP 0 630 652 A2

This invention relates to the use of molecular sieves as contrast agents in diagnostic imaging procedures and in particular in magnetic resonance imaging (MRI).

Contrast agents may be administered in medical imaging procedures, for example X-ray, magnetic resonance and ultrasound imaging, to enhance the image contrast in images of a subject, generally a human or non-human animal body. The resulting enhanced contrast enables different organs, tissue types or body compartments to be more clearly observed or identified. In X-ray imaging the contrast agents function by modifying the X-ray absorption characteristics of the body sites in which they distribute; magnetic resonance contrast agents generally function by modifying the characteristic relaxation times $T_1$ and $T_2$ of the nuclei, generally water protons, from the resonance signals of which the images are generated; and ultrasound contrast agents function by modifying the speed of sound or the density in the body sites into which they distribute.

The X-ray contrast agents first developed, barium sulfate and sodium iodide, have been superseded by iodinated organic compounds, in particular triiodophenyl compounds. Improvements in systemic toxicity over the last 40 years have also been achieved by the development of non-ionic iodinated X-ray contrast agents (see Shaw in "Radiopaques", CRC Handbook of Vitamins, Hormone and Radiopaques, CRC Press, p. 229-243).

As the X-ray absorption cross-sections of the elements generally increase with increasing atomic number and as such cross-sections are dependent on the wavelength of the X-rays there has been some desire to utilize the X-ray absorption properties of the lanthanides and other high atomic number metals to develop contrast agents with improved X-ray attenuation especially at the wavelengths used in CT; however these attempts have generally been relatively unsuccessful.

Thus, for example, Nalbandian et al. (see Ann. N.Y. Acad. Sci. 78: 779 (1959)) and Shapiro et al. (see Ann. N.Y. Acad. Sci. 78: 756 (1959)) proposed the use of the diethylenetetraaminepentaacetic acid (DTPA) chelate of bismuth (BiDTPA) and the ethylenediaminetetraacetic acid (EDTA) chelate of lead (PbEDTA) as radiographic contrast agents but encountered problems of solubility and toxicity. In US-A-4176173 Winchell et al. described the use of simple hafnium or tantalum complexes as X-ray contrast agents and, more recently, ytterbium DTPA has been studied as an intravascular X-ray contrast agent and an $LD_{50}$ of 10 mmoles/Kg has been reported (see Unger et al. Invest. Radiol. 21: 802 (1986)).

In MRI, the use of paramagnetic metal ions, such as Mn(II), as contrast agents was first proposed by Lauterbur et al. in 1978 (see pages 752-759 in "Electrons to Tissues - Frontiers of Biological Energetics" Vol. 1, edited by Dutton et al., Academic Press, NY, 1978) and more recently Schering AG in US-A-4647447 proposed the use of salts of gadolinium(III) chelates of DTPA.

In order to achieve tissue-specific MRI contrast enhancement or to enhance relaxivity the coupling of paramagnetic chelates, such as GdDTPA, or metal complexing groups to macromolecular carriers or biomolecules, such as polysaccharides, proteins, antibodies, liposomes, enzymes, polyethyleneimines etc. has been proposed by several researchers - see for example EP-A-130934 (Schering), EP-A-136812 (Technicare), EP-A-184899 (Nycomed), EP-A-186947 (Nycomed), EP-A-277088 (Schering), EP-A-305320 (Schering), WO-A-88/07521 (Schering), WO-A-88/08422 (Schering), WO-A-85/05554 (Amersham), WO-A-89/06979 (Nycomed), EP-A-331616 (Schering) and Schmiedl et al. Radiology 162:205 (1987).

For the visualization of the gastronitestinal (GI) tract however metal chelate contrast agents are generally unsuitable because of instability or side effects. Thus for example the commercially available MRI contrast agent GdDTPA produces relatively poor contrast improvement in the GI tract while causing diarrhoea (see Caussen et al. ROFO 148: 683 1989)). Nonetheless several insoluble GI tract MRI contrast agents have been proposed such as the gadolinium oxalate particles of Runge (US-A-4615879) and the superparamagnetic particles of Jacobsen (US-A-4863715). Recently Balkus et al (ACS National Meeting, Atlanta, Georgia, 14-19 April 1991) have proposed the use as GI tract MRI contrast agents of zeolites which have been loaded with paramagnetic ions such as gadolinium by cation exchange, or which have paramagnetic metal atoms such as iron or mangenese incorporated into the oxide framework by substitution of framework aluminiums. Balkus also suggested that zeolites similarly loaded with other heavy metals could also be used as GI tract X-ray contrast agents.

Zeolites are a set of aluminosilicate molecular sieves which have cation exchange properties due to the occurrence of trivalent aluminium in the molecular sieve oxide framework which leads to the presence of anionic cation-binding sites. There are silicon dioxide analogues to the zeolites but since these contain only oxygen and tetravalent silicon in the oxide framework the overall framework charge is zero and these molecular sieves are not cation exchangers. It is known that metal cations can be substituted for some of the trivalent aluminiums in a zeolite framework. These are generally trivalent cations having an ionic radius similar to that of the trivalent aluminium. As a result the framework charge is unchanged and the resultant molecular sieve retains its cation exchange properties. In certain cases metal cations can also be

substituted for framework silicons and, for the same reasons, the product can remain an ion exchange material.

There are also aluminophosphate molecular sieves (AlPOs) which are analogous to the silicon dioxides and, like the silicon dioxides the framework is uncharged so that the materials are not ion exchangers. As with the zeolites it is possible to substitute metal or other atoms for AlPO framework atoms, for example to produce the so called SAPO, MeAPO, MeAPSO, ElAPO and ElAPSO molecular sieves. The MeAPOs are isomorphous with the AlPOs, and with the metal (Me) substituting for framework aluminiums with the 1:1 metal (i.e. Al alone or Al + Me) to phosphorus ratio being retained.

Molecular sieves, and in particular the aluminosilicates and aluminophosphates, are widely used as catalysts and for separation procedures. Their useful properties derive primarily from their own structures with cavities and channels connected by uniform pores of approximately molecular dimensions (about 0.2 to 2nm).

In the cation exchanger zeolites these structures include regular framework anion sites and as a result the zeolites can be loaded with paramagnetic or heavy metal ions by relatively simple ion exchange procedures. In such loaded zeolites the paramagnetic metals are accessible to water molecules, which are relatively small and so can penetrate the lattice, and as a result the metal ion retains its ability to function as an MRI $T_1$ contrast agent. For a $T_2$ or $T_2^*$ contrast agent, access by water to the paramagnetic metal ion is not required. The pores however are too small to permit many of the other biomolecules encountered in the GI tract to enter and thus the sieves' structure serves to shield the metal ions from the body fluids.

Aluminosilicate molecular sieves have long been used as food additives. Thus as carrier matrices for paramagnetic or heavy metal ions for administration to human or animal subjects undergoing diagnostic investigation, the molecular sieves by themselves generally do not pose severe problems of toxicity.

Balkus further suggested chelation of the paramagnetic metal ions within the zeolite as a means by which metal loss from the zeolite might be reduced and metal location within the zeolite might be optimized - the dimensions of the complexes are such that these must reside in the supercages or large pores of the zeolites, the sites to which water molecules will have the easiest access. In this regard Balkus suggested the use of a range of bulky aromatic acidic (anion forming) chelants. Balkus also referred in this context to the possibility being available to form the chelate complexes in situ, as discussed for example in his earlier work on the production of zeolite encapsulated metal chelates, ie. the so-called ship-in-a-bottle chelates. (See for example Balkus et al J. Inclusion Phenom. 10: 141-151 (1991), Zeolites 10:722-729 (1990). JCS Chem Comm 57-58 (1991) and J. Phys Chem 94: 8019-8020 (1990)).

An alternative approach proposed by Balkus was a modification of the so-called template synthesis. It is well known that the synthesis of molecular sieves may require a directing ion or a so-called template. Directing ions are generally alkali metal or alkaline earth metal ions supplied to the synthesis preparation as a salt or base. The templates are typically organic molecules such as amines or polyamines supplied as the free amine, as a quaternary salt or as a quaternary base. The directing ion or template, the nature of the reactants, the pH and reaction conditions all have a profound impact on the structure, composition and properties of the molecular sieve that is formed.

In the case of template directed syntheses, the template may become trapped in the channels or cages of the sieve that is formed and in order to take advantage of the porosity of the sieve in for example catalytic or sorption applications thereof the trapped template must be removed. This is conveniently achieved by a high temperature combustion process referred to as calcination.

Balkus however has proposed that paramagnetic metal amine coordination complexes be used as templates in zeolite synthesis, that the calcination step be omitted and that the resulting zeolite encapsulated paramagnetic complexes be used as MRI contrast agents. (See for example "Teaching an old crystal new tricks", pages 5-7 of WPI-Journal, Summer 1991, Worcester Polytechnic Institute).

The bulky complexes proposed by Balkus however may serve to hinder water access to the metal coordination sphere and Balkus himself acknowledged that a pore blockage problem may exist and that full chelation of the paramagnetic species might be undesirable.

Thus there remains a need for improved molecular sieve encapsulated contrast agents and in particular such agents with reduced potential for releasing paramagnetic or heavy metal ions in vivo.

The present invention results in part from the recognition that modification of the surface of the molecular sieve particles may serve to reduce the risk of metal ion release in vivo.

Thus viewed from this aspect the invention provides a contrast agent composition for use in a method of diagnostic imaging, said composition comprising a physiologically tolerable carrier or excipient together with a particulate, diagnostically effective metal containing, surface-modified molecular sieve, said diagnostically effective metal being for example a paramagnetic or radioactive ion or atom or having an atomic number of at least 20, preferably at least 37, especially preferably at least 50.

The molecular sieves used according to this aspect of the invention are preferably cation exchangers and especially preferably zeolites. The surface modification according to the invention generally serves one or both of two purposes - the reduction in the available cation binding sites on or near the particle surfaces and thus the reduced leaching of diagnostic metal from such exposed sites following administration, and the constriction of pore mouth sizes so as to prevent loss of diagnostic metal from within the particles without preventing access of ambient water molecules, access which is of course important where the diagnostic metal is paramagnetic and the contrast agent compositions are MRI $T_1$ contrast media. Surface modification can also assist in disguising the molecular sieve particles from immunogenic response following parenteral administration.

In a further aspect of the invention, the diagnostically effective metal is present within the molecular sieve particles in the form of an encapsulated cationic chelate complex. Using encapsulated cationic complexes according to the invention the risk of metal loss from the molecular sieve in vivo, and thus the risk of potentially harmful uptake of the metal by the organism under investigation is reduced because of the attraction between the complex as a whole and the anionic sites on the molecular sieve framework. This can be achieved using chelating agents which are electrically neutral or have a lower negative charge than the positive charge on the diagnostically effective metal ion loaded into the molecular sieve cavity. However in a preferred aspect of this invention one may use a chelating agent having one or more (e.g. 1, 2 or 3) cationic or cation forming moieties, for example positively charged moieties, e.g. pendant ammonium groups, which are capable of direct interaction with the anionic cation binding sites on the molecular sieve framework or uncharged but basic moieties, such as pendant amine groups which can combine with partially cation exchanged acidic forms of zeolites, e.g. to generate an ammonium moiety in situ.

Such pendant cationic or cation forming functional groups achieve what amounts to a cross-linking effect binding the chelate complex more tightly to the molecular sieve carrier matrix.

The molecular sieve encapsulated chelate complex contrast agents in which the chelant is cationic or has a framework binding cationic functional group are novel and they and contrast media (compositions) containing them form further aspects of the present invention.

The range of chelating agents suitable for use according to the invention is large and well described within the scientific and patent literature. The bipyridyl, polyamine and aminopolycarboxylic acid chelating agents represent three well known groups which are suitable for this use. Ideally the chelating groups will themselves be uncharged groups capable of forming only dative bonds but chelating agents having small numbers (e.g. 1, 2, 3, 4 or 5 but generally less than 10) of anionic chelating groups may be used as long as the overall complex is itself cationic. Thus for the aminopolycarboxylic acid chelating agents it is desirable that at least some of the carboxy functions be derivatized to reduce their anion-forming tendency, e.g. by reduction, amidation or esterification or a combination thereof. It is particularly desirable that chelating agents having anion forming groups should also have cationic groups which will serve to ensure that the overall complex is cationic and may be available for binding to the molecular sieve framework as discussed above.

It is also preferred that the chelating agents not be such as to occupy all of the coordination sites on the complexed metal ion. This will ensure ready metal coordination by water molecules. This of course is important where the metal-carrying molecular sieve is to be used as a $T_1$ MRI contrast agent rather than where it is to be used in another imaging modality. Especially preferred ligands include the neutral or even positively charged (cationic) ligands as it is preferred that the overall complex carry as high a positive charge as possible. Thus specific examples of suitable chelating agents include for example EDA (ethylenediamine), bipy (bipyridyl),

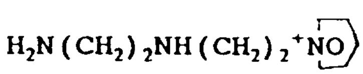,

ammonium nitriles and the like. Due to spatial constraints, the chelating agents used will generally be essentially linear molecules although molecules capable of joining up within a molecular sieve supercage to produce a macrocyclic ligand may also be used, i.e. the cationic complex may be generated in situ by template production.

In situ production of ship-in-a-bottle zeolite encapsulated complexes has been discussed for example by Balkus et al. in J. Incl. Phenom. 10:141-151 (1991). The preparation of encapsulated paramagnetic metal chelates is also described for example by Mikheikin et al. Russian Chem. Revs 41:468-483 (1972), Seff Accounts of Chem. Res. 9:121-128 (1970) and Lundsford Catal. Rev. Sci. Eng. 12:137-162 (1975).

Molecular sieve encapsulated chelate complexes of heavy metal ions are particularly useful in diagnostic imaging or therapy. Especially preferred are complexes with metals of atomic numbers 20-32,

42-44, 49 and 57 to 83, especially Fe, Mn, Gd, Dy and Yb. For use as an MR-diagnostics contrast agent, the chelated metal species is particularly suitably a paramagnetic species, the metal conveniently being a transition metal or a lanthanide, preferably having an atomic number of 21-29, 42, 44 or 57-71. Metal chelates in which the metal species is Eu, Gd, Dy, Ho, Cr, Mn or Fe are especially preferred and $Gd^{3+}$, $Mn^{2+}$ and $Dy^{3+}$ are particularly preferred.

For use as contrast agents in MRI, the paramagnetic metal species is conveniently non-radioactive as radioactivity is a characteristic which is neither required nor desirable for MR-diagnostics contrast agents. For use as X-ray or ultrasound contrast agents, the chelated metal species is preferably a heavy metal species, for example a non-radioactive metal with an atomic number greater than 37, preferably greater than 50, e.g. $Dy^{3+}$.

For use in scintigraphy and radiotherapy, the chelated metal species must of course be radioactive and any conventional complexable radioactive metal isotope, such as $^{99m}Tc$ or $^{111}In$ for example, may be used. For radiotherapy, the chelated metal may be for example $^{153}Sm$, $^{67}Cu$ or $^{90}Y$.

Molecular sieves are discussed at length by Szostak in "Molecular sieves" Van Nostrand Reinhold, NY, 1989. The molecular sieve used according to the invention may be any one capable of retaining the paramagnetic/heavy/radioactive metal within its structure. However the materials which have anionic cation-binding sites are especially preferred, in particular aluminosilicates (especially zeolites), as are the aluminophosphates (especially the so called MeAPO molecular sieves). Moreover molecular sieves having channels in more than one direction will generally be preferred relative to those only having channels running in one direction. Thus the three dimensional (e.g. beta) and caged (e.g. X and Y) zeolites are of particular interest. This is because the cross sectional area at channel intersections is greater than in the channels with the result that complexes larger than the channels may be held relatively firmly at these intersections with their release being sterically inhibited.

In a further aspect the invention provides a process for the preparation of a molecular sieve contrast agent according to the invention, said process comprising sequentially or simultaneously: (a) loading a particulate molecular sieve with a diagnostically effective metal; and (b) modifying the surface of the particles of said molecular sieve, e.g. by dealumination and calcination, by pore mouth engineering, etc. as described further below.

Where the metal containing molecular sieve is to be used as an MRI contrast agent it is of course important that water should be able to penetrate its structure and accordingly sieves with a pore size (i.e. channel diameter or cage:cage opening diameter as appropriate) less than 0.4 nm will not generally be used as water diffusion rates may be undesirably low.

Where the metal carrying molecular sieve is to be used as a GI contrast agent, it should of course be in a form adequately stable for such use. Thus in the stomach the ambient pH is strongly acid while in the intestines the pH rises to near neutral. Many molecular sieves are unstable in acidic media giving rise to materials with increased meso-porosity through the dissolution and removal of framework aluminium. Post-administration loss of aluminium and framework disintegration can of course lead to release of the metal chelate and thus is highly undesirable.

While this problem can be approached in a variety of ways, e.g. by administration of the molecular sieve in a "delayed release" form, e.g. within capsules that disintegrate only after passage through the stomach, this procedure is not appropriate for MRI contrast agents which are to be used for imaging the stomach. Thus for $T_1$ imaging the encapsulation prevents the necessary water access, and for $T_2$ imaging encapsulation prevents the required uniform distribution of the contrast agent. However the surface modification according to the invention can address the problem by having the modification serve to enhance the molecular sieve's acid resistance. Thus surface deactivation of cation-binding sites may be effected by dealumination of a zeolite, preferably preferential dealumination of the surface. In general, higher Si:Al ratios in molecular sieves such as zeolites lead to higher acid stabilities. However as the Al content is lowered the number of ion exchange sites drops and the maximum loading of the paramagnetic/heavy/radioactive metal is lowered. Moreover the hydrophilicity of the sieve decreases.

The Si:Al ratio can be adjusted to achieve the desired acid stability by selection of appropriate synthesis conditions or, more preferably, by post synthesis dealumination, e.g. steam treatment followed by acid treatment, acid treatment alone, EDTA treatment, $SiCl_4$ treatment, etc.

Dealumination of acidic crystalline molecular sieve materials can be achieved by exposing the solid material to inorganic acids such as $Gd(NO_3)_36H_2O$, HCl, $HNO_3$ and $H_2SO_4$, or to organic acids such as $CF_3COOH$ or inorganic-organic acids such as $CF_3SO_3H$. The use of inorganic acids however is preferred. The desired degree of dealumination will dictate the strength of acid used and the time during which the crystalline structure is exposed to the acid. Nevertheless, one desired method of dealumination is via steam treatment followed by a mild acid treatment and calcination or by steaming or mild acid treatment alone.

The steam treatment parameters are as follows:

| Parameter | Range | Preferred | More Preferred |
|---|---|---|---|
| Temperature °C | 200-1000 | 300-700 | 400-600 |
| Total Pressure, ATM | 0.001-15 | 0.001-5 | 0.2-2 |
| Carrier Gas | Flowing Gas | Inert ($N_2$, $H_2$) | |
| Length of Time | 0-24 hrs | 10 min. -4 hr. | 30 min. -1hr. |
| No. of Cycles | Several | 1-2 | 1-2 |
| Steam Partial Press. ATM | 0.001-15 | 0.001-5 | 0.1-2 |

After the steam treatment is completed, or initially if acid treatment alone is to be used, the next step should desirably be a mild acid treatment. The preferred acid treatment conditions are as follows:

| Parameter | Range | Preferred | More Preferred |
|---|---|---|---|
| Acid Type | -------- | Inorganic Acid | HCl, $HNO_3$ |
| Concentration | -------- | 0.01 - 2M | 0.1-1.0N |
| Temperature, °C | 10-100°C | 80-100°C | 100°C |

Additional methods of preparing aluminium-deficient zeolites are described by J Scherzer in "The Preparation and Characterization of Aluminium-Deficient Zeolites", Thaddeus E Whyte et al. in "Catalytic Materials: Relationship between Structure and Reactivity", at pp. 156-160, ACS Symposium Series 248, (American Chemical Society, 1984).

Dealumination according to the present invention is preferably so effected as to achieve a Si/Al ratio between 1 and 1000, preferably between 1 and 100, most preferably, 1 to 50.

Dealumination is also discussed by van Bekkum et al. "Introduction to zeolite science and practice", Elsevier, Amsterdam, 1991, pages 154 et seq., who describe how different dealumination techniques produce different materials and in particular how aluminium deficient surfaces can be produced using acid, steam and acid, and chelating agent (e.g. EDTA) treatments (see van Bekkum et al., page 172).

For the present invention, it is especially preferably that the zeolite is subjected to a calcination following the dealumination so that the hydroxyl nests left after aluminium removal are condensed so leaving the framework repaired. This can be shown schematically by the equation:

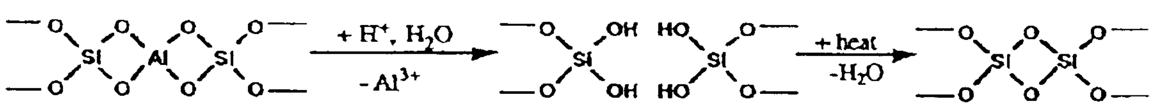


Thus a dealuminated crystalline molecular sieve can be dried at 200°C and calcined at temperatures between 400°C and 1000°C, preferably between 400°C and 600°C. Calcination serves to dehydrate or "heal" Si-OH bonds or "nests" after dealumination. Healing these nests provides for a more uniform pore structure within the crystalline material, leading to structural stability and ultimately resulting in improved water diffusion rates.

The molecular sieve particle size is also important whether the metal carrying particles are to be administered into the GI tract or elsewhere. The smaller the particle size the lower will be the tendency for the particles to settle or sediment and sedimentation is undesirable not least because it can lead to image artifacts. However as the particle size is decreased the ratio between the external surface area and the particle volume increases causing the acid stability problem to worsen and, perhaps much more significantly, increasing the relative proportion of cation binding sites which are on the exterior of the particle and are thus exposed to the ambient body fluids (relative that is to the number of cation binding sites which are within the molecular sieve's framework and so are shielded from the ambient fluids).

Furthermore, where the metal carrying molecular sieves are to be used as MRI contrast agents, water diffusion in and out of the particles is desirable for high $T_1$ relaxivity rates to be achieved. Accordingly the particle or crystallite size (e.g. weight average maximal dimension) should be selected to balance these criteria. Generally speaking however particle sizes of from 10 nm to 20 µm, especially 20 nm to 10 µm,

more especially 0.05 to 10 μm, e.g. 0.1 to 2 μm, are preferred.

The problem of increased external:internal cation binding site ratio with decreased particle size is significant as the potential for loss of the toxic metal by the molecular sieve in vivo increases. This problem is addressed in another aspect of the invention which involves the surface deactivation of the molecular sieve particles. Thus, the surface of the molecular sieve may be chemically modified, for example by cation-binding site deactivation or to enhance acid stability or to enhance dispersibility or biotargettability.

By surface deactivation of molecular sieve particles it is meant that surface anionic cation-binding (ion exchange) sites are modified to reduce or eliminate their tendency to bind metal cations, especially diagnostic cations. This can be achieved in several ways, for example by the adsorption of surface active reagents, e.g. using the acid form of the molecular sieve. This can be done before or after the particles are loaded with the diagnostic metal, preferably in chelate complex form and especially preferably in the form of cationic chelate complexes. However if deactivation occurs after loading, externally bound metal will have to be displaced and separated off. The surface deactivating agents are preferably sufficiently bulky as to hinder their passage into the interior structure of the molecular sieve and they may be selected so as to modify the surface properties of the particles, e.g. to minimize settling effects, to assist the formation of stable emulsions or to target the particles onto particular body sites. Examples of suitable reagents include silanes, silanols, titanates, phosphates, phosphonates, etc, which may be reacted with the molecular sieve followed if necessary by a calcination reaction.

Generally speaking, crystalline molecular sieves may be treated to modify internal and external cation exchange or acid sites by contact with a deactivating reagent selected from the group consisting of the halogen hydrotic and organic derivatives of Groups IIIA, IVA, IVB and VA. Preferred embodiments of the internal acid site deactivating reagents include $B_2H_6$, $SiH_4$ and $PH_3$. For a more complete discussion of the internal acid site modification techniques, see A. Thijs et al., J. Chem. Soc. Faraday Trans., 79, 2821 (1983). See also J Philippaerts et al., "The Implantation of Boron-Nitrogen Compounds in Mordenite LP and Their Influence on the Adsorption Properties," Stud. Surf. Sci. Catal., 28, 1986, pp. 305-310. The relevant portions of both references are hereby incorporated into this specification.

In addition to the use of the above described deactivating reagents, which tend to be non-specific, there is an intermediate level of crystalline molecular sieve modification which can be used to perform "pore mouth engineering" or surface modification. These reagents provide an intermediate level since they are not specific for external acid site, but are not entirely non-specific, leading to substantial internal acid site modification. In selecting an intermediate deactivating reagent, the characteristics and pore aperture dimensions of the starting crystalline molecular sieve must be matched against the molecular dimensions of the deactivating reagent.

It has been shown that chemical vapor deposition of $Si(OCH_3)_4$ on H-mordenite can be successfully used to control the intracrystalline pore aperture without substantially affecting the catalyst's internal surface acidic or cationic exchange properties. $Si(OCH_3)_4$ can be deposited irreversibly on zeolite, without entering the intra-crystalline pores. See Niwa, M et al., J. Chem. Soc., Faraday Trans., 1, 1984 80, 3135-3145; Niwa, M et al., "Modification of H-Mordenite by Vapour-phase Deposition Method, "J. Chem. Soc. Commun., 1982, pp. 819-20.

Similarly, chemical vapor deposition of deactivating metal chlorides such as $SiCl_4$, $GeCl_4$, $TiCl_4$ and $SnCl_4$ can be effective to modify pore mouth structures without inactivating internal surface acid sites. These metal molecules, with a range of molecular dimensions, can be selected to be larger than the catalyst pore aperture, thereby preventing substantial diffusion into the internal pore. See Hidalgo, C.V. et al., Zeolites, 1984 4, April, pp. 185-180.

It is also recognized that the deactivating agents can be contacted with the molecular sieve in either solution or vapor phase.

As noted above, it is desirable in any case to deactivate external surface cation exchange or acid sites, without regard to the pore aperture dimensions of the starting crystalline molecular sieve. External surface cation exchange or acid site deactivation can be obtained by either acid site blockage or acid removal. One major limitation of both techniques, however, is that the deactivating agent should be selected to preclude internal surface diffusion. This limitation is easily met by the use of deactivation agents in either liquid or gas phase, whose molecules are too large to fit within even the largest pores of known zeolites. One such molecule is triphenylchlorosilane. See Martens, J A et al., Zeolites, 1984, 4, April, pp. 98-100.

In addition to the elimination of surface cation exchange sites and to modify or enhance the surface properties of the zeolite, reactive agents such as described above can be used provided that the modifying species has at least one hydrolytically stable functional group which is biocompatible. Examples of such groups could include substituted polysaccharides (e.g. dextran), substituted sugars, peptides and polypeptides, etc.

Generally speaking, molecular sieves, preferably after loading with the diagnostic metal (e.g. by cation exchange) and optionally after dealumination and calcination, may be treated chemically to deactivate surface cation binding sites either by the use of hydrolytically unstable surface modifying reagents (such as metal alkoxides, hydrides and halides) or by the use of reagents having at least one hydrolytically stable moiety. Taking the example of silane reagents, reaction with $RSi(OR^1)_3$ where R is the stable moiety (e.g. an alkyl, aryl, aralkyl, alkaryl, etc group, for example having up to 20 carbons and optionally being substituted by one or more amino, hydroxy or carboxy groups) and $OR^1$ is an unstable group (where $R^1$ for example is defined as for R), the surface reaction may be illustrated schematically as follows:

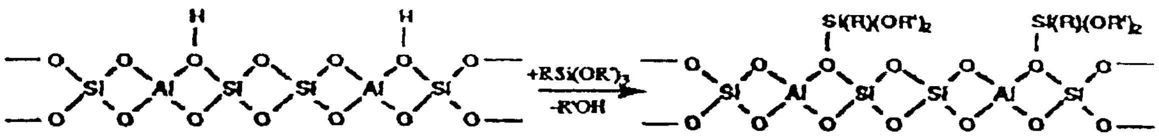


The substituent groups serve not only to deactivate surface cation binding sites and modify surface characteristics but also, due to their steric bulk, to constrict pore mouth openings.

Chemical treatment of the molecular sieve particles, especially after loading, can provide a further solution to the problem of mimimizing the risk of loss of toxic diagnostic metal from the particles; more specifically this solution involves pore mouth engineering so as to constrict the pore openings and thereby further hinder loss of encapsulated metal cations and more especially metal chelates. Viewed from this aspect the invention thus provides a diagnostic contrast agent comprising a particulate, diagnostic metal cation-encapsulating molecular sieve having constricted pore openings, i.e. constricted by pore mouth engineering or other chemical treatment.

Pore mouth constriction has a dramatic effect on the rates of diffusion of materials through the pores into and out of the particles and the technique may thus be used to further hinder passage out of the particle of the diagnostic metal, which is preferably in chelate form, particularly preferably in the form of cationic chelate complexes, without necessarily preventing rapid diffusion of water.

Pore mouth engineering (described for example by Vansant in "Innovation in zeolite materials science" (Ed. Grobet et al.), Elsevier, Amsterdam, pages 143-153 (ca. 1987)) can be achieved by coating the particle surface preferably with an inert material such as silica ($SiO_2$). This coating serves to achieve the twin desirable effects of deactivating surface ion exchange sites and, by virtue of the build up of the coating material around the pore mouth, reducing the diameter of the pore mouth opening and thereby slowing the diffusion rate through the opening for molecules larger than the new restricted size.

Using this technique it is feasible to utilize smaller chelating agents to achieve the same or better retention of the encapsulated metal complex so allowing better water access to the encapsulated complex within the molecular sieve framework. The technique can be used to adapt pore mouth openings with extreme accuracy, e.g. at the sub angstrom level, as is described by Niwa et al. in Chapter 3, "Design and preparation of catalyst", pages 255-286 in Proceedings of the International Symposium on Acid-Base Catalysis, Ed. Tanabe, Kodansha, Tokyo, 1989 (Chem Abs 113:175215c).

The surface modified, e.g. deactivated or pore size engineered, contrast agent is of course preferably formulated with one or more carriers or excipients to produce a contrast medium (composition) suitable for administration either directly or after further formulation (e.g. dilution, dispersion or suspension) and the contrast medium itself forms a still further aspect of the present invention.

For the relatively smaller diagnostic metals, i.e. atomic number less than 50, especially less than 35, most particularly Fe, Mn, Cr and Co, there is an alternative approach to chelate complex encapsulation or simple cation exchange which may be used to reduce the danger of diagnostic metal loss from molecular sieve particles. This involves the replacement of framework aluminium, silicon or phophorus atoms by atoms of the diagnostic metal. Thus replacement in zeolites of aluminium by chromium is discussed for example by Mikheikin (supra). However there is one class of molecular sieves, which are not ion exchangers and which are especially suitable in this regard. These are the so-called MeAPO aluminophosphates (see for example Wilson Studies in Surface Science and Catalysis No. 58, "Introduction to Zeolite Science and Practice" (Ed. van Bekkum et al.), Elsevier, Amsterdam, 1991, pages 137-151) in which MeAPOs incorporating Fe, Mn and Co are discussed. These materials are inherently paramagnetic and in some cases have coordination sites open for water coordination of the paramagnetic metal. Moreover the amount of diagnostic metal which can be loaded into a MeAPO structure in this way is greater than the amount that can be achieved with a zeolite for which the loading level may be insufficient to achieve fully

satisfactory relaxivities. The MeAPOs, either template synthesized or calcined forms, are also readily accessible and well characterized.

Thus in a still further aspect the invention provides the use of a MeAPO incorporating as a framework metal a divalent paramagnetic or radioactive metal species having an atomic number of less than 50, especially preferably less than 35, particularly a paramagnetic first row transition metal species, especially Co, Fe or Mn, for the manufacture of a diagnostic imaging contrast medium.

The MeAPOs useful according to this aspect of the invention may for example be prepared using template-based syntheses, with organic molecules such as polyamines used as the templates and with subsequent calcination as described above to rid the sieve of the entrapped template molecules. A modification of the Balkus technique of using paramagnetic metal ion (e.g. Mn, Gd etc.) complexes as the templates and then omitting the calcination step however offers a particularly favourable route to maximizing the paramagnetic loading of the MeAPOs.

Thus viewed from a still further aspect the invention provides a template synthesized MeAPO incorporating as a framework metal a divalent paramagnetic or radioactive metal species having an atomic number of less than 50, especially preferably less than 35, particularly a paramagnetic first row transition metal species, especially Co, Fe or Mn, and further encapsulating paramagnetic metal complex template residues.

The MeAPO particles may be surface treated as discussed above if desired and the appropriate particle sizes will be selected from similar ranges.

As discussed above, the contrast agents are intended for use in diagnostic imaging and thus in a further aspect the invention also provides a method of diagnostic imaging of a human or non-human animal (preferably mammalian) subject, said method comprising administering to said subject, preferably into the GI tract thereof, a molecular sieve contrast agent composition according to the invention or contrast medium produced according to the invention, and generating an image of at least part of said subject, e.g. by scintigraphy or ultrasound but preferably by MRI or X-ray imaging.

The diagnostic agents of the present invention may be formulated with conventional pharmaceutical or veterinary formulation aids, for example stabilizers, antioxidants, osmolality adjusting agents, buffers, pH adjusting agents, etc. and may be in a form suitable for parenteral or enteral administration, for example injection or infusion or administration directly into a body cavity having an external escape duct, especially the gastrointestinal tract (e.g. by oral or rectal administration, for example as an orally ingestible suspension or as an enema), the bladder or the uterus. Thus the compositions of the present invention may be in conventional pharmaceutical administration forms such as tablets, capsules, powders, suspensions, emulsions, dispersions, syrups, suppositories, etc; however suspensions and dispersions in physiologically acceptable carrier media, for example water or saline, will generally be preferred.

The agents according to the invention may therefore be formulated for administration using physiologically acceptable carriers or excipients in a manner fully within the skill of the art. For example, the loaded molecular sieves, optionally with the addition of pharmaceutically acceptable excipients, may be suspended in an aqueous medium, with the resulting suspension then being sterilized. Suitable additives include, for example, physiologically biocompatible buffers (as for example, tromethamine hydrochloride), additions (e.g., 0.01 to 10 mole percent) of chelants (such as, for example, DTPA or DTPA-bisamide) or calcium chelate complexes (as for example calcium DTPA, CaNaDTPA-bisamide), or, optionally, additions (e.g. 1 to 50 mole percent) of calcium or sodium salts (for example, calcium chloride, calcium ascorbate, calcium gluconate or calcium lactate).

If the agents are to be formulated in suspension form, e.g. in water or physiological saline for oral administration, they may be mixed with one or more of the inactive ingredients traditionally present in oral solutions and/or surfactants and/or aromatics for flavouring.

Particularly preferred components for the contrast media compositions also include gelling agents and in particular clays, for example smectite clays such as montmorillonite. The clays used can be diagmagnetic or paramagnetic (either naturally so or by ion exchange with paramagnetic cations). The weight ratios of such molecular sieve:clay combinations will preferably be such that 1 part by weight molecular sieve is combined with up to 100 parts by weight of clay.

For oral MRI contrast media it is of particular interest to provide a composition which can function effectively as both a $T_1$ and a $T_2$ agent. Compositions effective in this regard can be achieved by the combination of a paramagnetic metal-loaded molecular sieve such as those discussed above (e.g. a zeolite encapsulated paramagnetic metal complex, preferably a cationic complex of gadolinium, or a MeAPO in which the framework substitution metal Me is paramagnetic, e.g. Co, Fe etc) with a further relaxation rate enhancing agent such as a particulate superparamagnetic, ferromagnetic or ferrimagnetic material, a clay (especially a smectite clay such as montmorillonite) or a fluorinated (preferably perfluorinated) agent, e.g.

PFOB.

Alternatively and desirably the molecular sieve may be loaded with two or more different paramagnetic species having different $T_1/T_2$ effects, e.g. by using a combination of gadolinium and dysprosium. This may be achieved very simply by substituting $Dy(NO_3)_3$ for a small proportion of the $Gd(NO_3)_3$ used in solution to load a molecular sieve by cation exchange.

Fluorinated agents, metal oxides, especially ferrites, and other superparamagnetic, ferromagnetic and ferrimagnetic materials suitable for use in MRI contrast media, particularly media administrable into the GI tract are well known and have been widely discussed in the patent and technical literature, e.g. WO-A-89/11873 (Klaveness), EP-B-186616 (Gries), EP-A-368429 (Blaszkiewicz), US-A-4951675 (Groman), and the documents cited therein.

Thus clays can perform a dual function in such combined media - as gelling agents and as relaxation agents. Similarly the perfluorinated agents such as PFOB can also perform not only as relaxation agents but also to increase the bowel transit time of the ingested contrast medium.

Accordingly, viewed from a further aspect, the invention provides an MRI Contrast medium (composition) comprising a paramagnetic metal species containing molecular sieve together with at least one relaxivity enhancing agent selected from the group consisting of physiologically tolerable clays, fluorinated organic compounds, ferromagnetic, ferrimagnetic and superparamagnetic particles, the magnetic particles optionally being carried or aggregated by a physiologically tolerable matrix material, said composition optionally further comprising at least one physiologically tolerable carrier or excipient.

For MRI and for X-ray imaging of some portions of the body the most preferred mode for administering the contrast agents is parenteral, e.g. intravenous, administration. Parenterally administrable forms, e.g. intravenous suspensions, should be sterile and free from physiologically unacceptable agents, and should have low osmolality to minimize irritation or other adverse effects upon administration, and thus the carrier medium should preferably be isotonic or slightly hypertonic. Suitable vehicles include aqueous vehicles customarily used for administering parenteral solutions such as Sodium Chloride Injection, Ringer's Injection, Lactated Ringer's Injection and other solutions such as are described in Remington's Pharmaceutical Sciences, 15th ed., Easton: Mack Publishing Co., pages 1405-1412 and 1461-1487 (1975) and The National Formulary XIV, 14th ed. Washington: American Pharmaceutical Association (1975). The suspensions can contain preservatives, antimicrobial agents, buffers and antioxidants conventionally used for parenteral solutions, excipients and other additives which are compatible with the molecular sieves and which will not interfere with the manufacture, storage or use of the products.

Where the diagnostic agent comprises a toxic metal species, e.g. a heavy metal ion, it may be desirable to include within the formulation a slight excess of a chelating agent, e.g. as discussed by Schering in DE-A-3640708, or more preferably a slight excess of the calcium salt of such a chelating agent. For MR-diagnostic examination, the diagnostic agent of the present invention, if in suspension or dispersion form, will generally Contain the diagnostic metal at concentrations in the range of 1 micromole to 1.5 mole per litre, preferably 0.1 to 700 mM. The diagnostic agent may however be supplied in a more concentrated form for dilution prior to administration. The diagnostic agents of the invention may Conveniently be administered in amounts of from $10^{-3}$ to 3 mmol of the diagnostic metal species per kilogram of body weight, e.g. about 1 mmol Gd/Kg bodyweight.

For X-ray examination, the dose of the contrast agent should generally be higher and for scintigraphic examination the dose should generally be lower than for MR examination.

The disclosures of all the documents mentioned herein are incorporated by reference.

The invention will now be described further by way of illustration with reference to the following non-limiting Examples:

Si/Al ratios were obtained using ICP analyses. Surface area data were obtained using BET nitrogen absorption measurements.

Solid state MAS (magic angle spinning) and CPMAS (cross polarization magic angle spinning) NMR can be particularly useful in determining the effectiveness of calcination. Calcination serves to dehydrate or "heal" Si-OH bonds or "nests". Healing- these nests provides for a more uniform pore structure, which improves selectivity, and also gives a more stable structure. From the CPMAS spectra only those silicon atoms which were in close proximity to protons were observed. Typically, this requires that the silicon be either a siloxyl silicon or within a few angstroms of a siloxyl group. Thus, the intensity of the silicon resonance is a direct measurement of the amount of Si-OH bonds present in the zeolite. As the calcination temperature is increased, the intensity of the peak decreases. The CPMAS spectrum of a dealuminated mordenite calcined at 400 °C exhibits a noticeable silicon resonance indicating a significant amount of Si-OH nests remaining in the struture. The CPMAS spectrum of a similar mordenite calcined at 500 °C contains only a negligible peak which indicates a near absence of Si-OH nests.

EXAMPLE 1

Ten grams of Toyo Soda H-Mordenite was loaded in a fritted quartz tube and placed vertically in a tube furnace. A slow helium flow of about 200 $cm^3$/min was introduced at a flow rate of about 1.5 litres/minute. After thirty minutes the steam was discontinued, the helium flow was lowered to about 200 $cm^3$/min and the furnace cooled to ambient temperature. The solid was then transferred to a 500 mL round-bottom flask equipped with a condenser and a stir bar. Two hundred millilitres of 0.5 N hydrochloric acid was introduced and the resultant mixture refluxed for four hours. After cooling, the solution was filtered and washed with distilled water until the filtrate was free of chloride ions by a $AgNO_3$ test. The solid was first dried at 110°C for two hours then calcined at 500°C for eight hours.
If desired, the solid can be subjected to a number of steam calcination and acid washing cycles. After drying the solid at 110°C the solid can be re-introduced to the fitted quartz tube for an additional steam calcination.

EXAMPLE 2

Toyo Soda and Norton H-Mordenite were dealuminated following a procedure similar to that of Niwa (Chem Lett. 1987, 1637). Five grams of zeolite were dealuminated with a hydrochloric acid soution at 93°C. After dealumination, the solution was filtered and the solid washed with distilled water until no chloride was detected with a $AgNO_3$ solution, followed by drying at 120°C and calcining at 500°C for one hour.

EXAMPLE 3

Three samples of synthetic H-Mordenite were dealuminated using a procedure similar to that shown in Example 2, i.e. the zeolites were steamed, leached in 0.5 N hydrochloric acid for four hours, and calcined in air. The particulars of the specific dealumination steps are as follows:

| Example No. | Steam **@ To | Calcination |
|---|---|---|
| 3(a) | Steam in He @ 400°C | 500°C for 12h in air |
| 3(b) | Steam in He @ 400°C | 500°C for 16h in air |
| 3(c) | Steam in He @ 600°C | 500°C for 8h in air |

* The acid treatment step took place between steaming and calcination steps.
* * Each steaming step was 30 minutes long.

Each of the dealuminated samples was then measured for Si/Al ratio using the methods discussed above. The values are as follows:

| Example No. | Si/Al Ratio |
|---|---|
| 3(a) | 19 |
| 3(b) | 25 |
| 3(c) | 30 |

EXAMPLE 4

The method of dealumination used in this example generally follows the procedure disclosed in Example 1 of EP-A-317907.
The first sample (a Toyo Soda Na-Mordenite) was exchanged with 1N-HCl for a period of 30 minutes, rinsed, and calcined for two hours in an oven at 700°C. The treated sample is Example 4 (a). The procedure produced a zeolite having an Si/Al ratio of 14.
The second sample began as Example 4 (a). It was dealuminated by refluxing the material under 6N-$HNO_3$ for two hours. The material was washed, dried, and calcined in an oven at 700°C for two hours. This procedure gave dealuminated material (Example 4 (b)) having a Si/Al ration of 63.
The third material began as a Toyo Soda H-Mordenite. It was calcined in air at 700°C for two hours, refluxed in 6N-$HNO_3$ for two hours, washed, rinsed, and again calcined in air at 700°C for two hours. This

procedure resulted in a material (Example 4 (c)) having a Si/Al ratio of 120.

EXAMPLE 5

A sample of Na-Mordenite was exchanged twice with an appropriate amount of 5N $NH_4Cl$. After draining and rinsing, the material was steamed for 30 minutes with 400 ° C steam and acid leached with 0.5N HCl for four hours. Finally, the washed mordenite was calcined for 12 hours at 500 ° C in air. The resulting material had a Si/Al ratio of 17.3.

EXAMPLE 6

6 a. Ion exchange of Zeolite A with $Gd^{3+}$

Approximately 1.3 M solution of gadolinium nitrate in water was prepared by dissolving 30 g of $Gd(NO_3)_3 6H_2O$ in distilled water with slight heating. To 50 mL of the gadolinium solution was added 10g of Zeolite A ($Na_{12}[(AlO_2)_{12}(SiO_2)_{12}]*27H_2O$). This mixture was refluxed for 6 hours. After refluxing, the ion-exchanged zeolite was obtained by centrifugation for 5 min at 3500 rpm. The zeolite was washed with distilled water and the wash was discarded by decanting after centrifugation. The ion-exchanged zeolite was washed by this procedure of centrifugation and decanting a total of three times. After the third wash, the ion-exchanged zeolite was placed in a porcelain crucible and calcined at 500 ° C overnight in a muffle furnace. The procedure of refluxing, washing and calcining was performed a total of three times. The final yield was 9.8g. Elemental analysis was conducted on the sample. The sample stoichiometry was $Gd_{3.6}Na_{1.3}[(AlO_2)_{12}(SiO_2)_{12}]$ which represents a 90% theoretical loading of Gd.

6 b. Ion exchange of Zeolite Y with $Gd^{3+}$

Approximately 1.3 M solution of gadolinium nitrate in water was prepared by dissolving 30 g of $Gd(NO_3)_3 6H_2O$ in distilled water with slight heating. To 50 mL of the gadolinium solution was added 10g of Zeolite Y ($Na_{56}[(AlO_2)_{56}(SiO_2)_{136}]*250H_2O$). This mixture was refluxed for 6 hours. After refluxing, the ion-exchanged zeolite was obtained by centrifugation for 5 min at 3500 rpm. The zeolite was washed with distilled water and the wash was discarded by decanting after centrifugation. The ion-exchanged zeolite was washed by this procedure of centrifugation and decanting a total of three times. After the third wash, the ion-exchanged zeolite was placed in a porcelain crucible and calcined at 500 ° C overnight in a muffle furnace. The procedure of refluxing, washing and calcining was performed a total of three times. The final yield was 8.9g. Elemental analysis was conducted on the sample. The sample stoichiometry was $Gd_{14.7}Na_{5.9}[(AlO_2)_{50.0}(SiO_2)_{136.0}]$ which represents a 88% theoretical loading of Gd.
The gadolinium solution used for cation exchange according to Example 6(b) is a weak inorganic acid and caused dealumination of the zeolite. Approximately 10% of the aluminium was lost (i.e. Si/Al ratio = 2.7).

6 c. Ion exchange of Zeolite-Y with $Gd^{3+}$

Na-Y is exchanged twice with 5N $NH_4Cl$. After filtering and washing with water until no further chloride is detected (using the Ag $NO_3$ test), the zeolite is ion exchanged with $Gd(NO_3)_3 6H_2O$ using the procedure of Example 6(b) to produce a product mixture containing $Na^+$, $NH_4^+$ and $Gd^{3+}$ forms. This product is calcined in air at 400 ° C for 2 hours and is converted to the $Na^+$, $H^+$ and $Gd^{3+}$ form.

EXAMPLE 7

Ion exchange of dealuminated molecular sieve

Dealuminated molecular sieves produced according to Examples 1 to 5 are loaded with gadolinium analogously to Example 6(b).

EXAMPLE 8

Dealuminated molecular sieves produced according to Examples 1 to 5 are loaded with gadolinium and dysprosium analogously to Example 6(a) with a 20% mole fraction of the $Gd(NO_3)_3$ replaced by $Dy(NO_3)_3$.

EXAMPLE 9

9a. Surface modification of paramagnetic metal loaded zeolite

The gadolinium loaded zeolite of Example 6(b) is treated with $SiCl_4$ (or $Si(OC_2H_5)_4$) for 1 to several hours at 200 to 600 °C. The product is cooled to ambient temperature and washed with water. It is then calcined in flowing air for four hours at 600 °C.

9b. Surface modification of paramagnetic metal loaded zeolite

The gadolinium loaded zeolite of Example 6(b) is treated with a large excess (ca. one thousand fold stoichiometric excess) of R $Si(OC_2H_5)_3$ (where R is $C_2H_5$, $CH_2Cl$, $CH_2CH_2NH_2$, $CH_2CH_2OH$ or $CH_2CH_2COOH$) either neat or in a hydrocarbon solvent such as hexane or $CH_2Cl_2$. The resultant product is washed with water and dried at 150 °C for 4 hours.

EXAMPLE 10

Oral administration form

| | |
|---|---|
| Gadolinium loaded zeolite (according to Example 6(a), 7, 8 or 9) | 1 g |
| EZ-paque supernatant | 99 ml |

The gadolinium-loaded zeolite is dispersed in the supernatant.

EXAMPLE 11

Oral administration form

The gadolinium-loaded zeolite of Example 6(b) is dispersed in agar to produce suspensions containing gadolinium concentrations of 0.07, 0.1, 1.0, 2.0 and 4.0 mM Gd.

EXAMPLE 12

Relaxivity and Phantom Studies

Relaxivity

The longitudinal and transverse relaxivity of the gadolinium-loaded zeolites of Examples 6(a) and 6(b) were determined in a 0.4% agar matrix. A sample Concentration range of 0.07 - 4 mM $Gd^{3+}$ was investigated. Three sample replicates at each concentration level were prepared and analyzed.
All relaxation times ($T_1$ and $T_2$) were determined at 37 °C and 0.47 T (20 MHz). The $T_1$ values were measured from twenty-five data points generated from an inversion recovery sequence. (An enhancement of one was used for all sample analysis.) A monoexponential three parameter fit was used to calculate the $T_1$, phase angle and maximum intensity values.
A modified CPMG SE sequence was used to measure the transverse relaxation times. The two values were determined at two different echo times: 4.0 and 10.0 ms (LC = 30). Raw data was fit by both a monoexponential and biexponential function (plus offset).
The particle volume diameter and specific surface area were determined by light diffraction with a Malvern Mastersizer 1002. The samples were diluted within 100 mL Isoton II. The Number concentration was determined by a Coulter Multisizer Mrk II.
The results are summarized in the table below:

| Sample | EW* | Volume Diam. (μm) | SP. Surface Area $m^2$/mL | No. Conc. $10^6$/mL | r1, $S^1mM^{-1}$ 0.4% agar | TE ms | r2, $S^1mM^{-1}$ | r1/r2 |
|---|---|---|---|---|---|---|---|---|
| 6(a) | 627.7 | 8.5 ± 0.0 | 1.3 ± 0.0 | 19 ± 1 | 1.21 ± 0.22 | 4 | 23.0 ± 0.8 | 0.05 |
| | | | | | | 10 | 19.6 ± 1.5 | 0.04 |
| 6(b) | 1082 | 6.0 ± 0.1 | 2.3 ± 0.0 | 65 ± 1 | 9.05 ± 0.08 | 4 | 36.2 ± 0.4 | 0.25 |
| | | | | | | 10 | 42.0 ± 2.1 | 0.21 |

* = The equivalent weight was determined from the empirical formulas obtained by ICP analysis.

The evolution of the transverse magnetization of both zeolites was biexponential as expected. (The multiexponentiality of the system is believed to be linked to water protons trapped within the zeolite and the bulk water protons which are free to migrate.) A biexponential evolution of the longitudinal magnetisation vector was also observed for the Example 6(a) product.

The relaxivity values for the Example 6(b) product are especially good. Its longitudinal relaxivity was 56% higher than that of Gd-DTPA in agar. (Note that two methods can be used to determine the in vitro effect of a contrast agent on a medium. The first implements the common contrast agent on a medium. The first implements the common contrast equation $C = (Ia-Ib)/(Ia + Ib)$ where I may equal the signal intensity, R1 or R2 of the medium and the second method, the r1/r2 ratio, which expresses the contrast agent's effect on both r1 and r2.) comparison of the products of Examples 6(a) and 6(b) shows that the Example 6(b) product has higher $r_1$ and $r_2$ values.

Phantom study

Seven concentration levels of each zeolite were prepared in 0.4% agar. The Phantoms were then prepared by filling 10 mL NMR tubes with the hot agar solution. The Phantoms were placed immediately in ice until the agar formed a stiff gel. The T1 and T2 relaxation times (at 0.5 T) were determined for all samples.

The Phantoms were imaged on both a T1-weighted spin echo (SE) and gradient echo (GE) sequence. The sequence parameters are summarized below:

| Parameter | Spin Echo | Gradient Echo |
|---|---|---|
| TR/TE | 450/12 | 120/7 |
| Flip Angle (°) | 90 & 180 | 60 |
| Averages | 2 | 8 |
| Scan Time (min) | 2 | 2 |

The results are summarized in the table below:

| Example 6(a) | | | | | Example 6(b) | | | | |
|---|---|---|---|---|---|---|---|---|---|
| Conc. mM Gd | SI (SE) | C % | SI (GE) | C % | Conc. mM Gd | SI (SE) | C % | SI (GE) | C % |
| 0 | 1.93±0.18 | - | 1.53 | - | 0 | 1.48±0.19 | - | 1.48 | - |
| 0.13 | 9.25±0.11 | 65.5 | 3.16 | 34.7 | 0.07 | 5.81±0.37 | 59.4 | 3.07 | 34.9 |
| 0.22 | 9.96±0.07 | 67.5 | 3.35 | 37.3 | 0.12 | 6.06±0.28 | 60.7 | 3.21 | 36.9 |
| 0.38 | 7.23±0.09 | 57.9 | 2.14 | 16.6 | 0.21 | 4.88±0.16 | 53.5 | 2.60 | 27.4 |
| 1.2 | 2.96±0.07 | 21.1 | 0.46 | -53.8 | 0.71 | 2.07±0.04 | 16.6 | 0.95 | -21.8 |
| 2.0 | 1.91±0.05 | -0.5 | 0.14 | -83.2 | 1.1 | 0.83±0.01 | -28.1 | 0.35 | -61.7 |
| 4.1 | 0.46±0.03 | -61.5 | 0.04 | -94.9 | 2.3 | 0.29±0.02 | -67.2 | 0.07 | -91.0 |

The "C" in the table above was calculated from the contrast equation:

$$C = (Ia-Ib)/(Ia + Ib)100.$$

Where: Ia is the signal intensity (SI) obtained from the phantom sample (post contrast),
Ib is the signal intensity of the blank sample (pre-contrast).

The "C" value is used to determine the change in signal intensity (as a %) caused by the addition of contrast agent to the agar matrix. (A positive "C" value represents an increase in signal intensity and a negative value reflects a decrease in signal intensity.)

The scaling factors are different for the GE and SE sequences, thus the signal values cannot be directly compared. The GE values are the mean of two slices while the spin echo results are the mean of 4 measurements (2 experiments with 2 slices each).

The GE sequence was more sensitive to susceptibility effects as illustrated by the increase negative "C" values for lower Gd concentrations. The product of Example 6(b) has a much larger susceptibility effect ($T_2$* contribution) than that of Example 6(a). At a concentration level greater than 0.7 mM Gd, the product of Example 6(b) caused an efficient reduction of signal intensity (GE sequence).

The optimal concentration ranges for signal enhancement are 0.07-0.12 mM Gd for the product of Example 6(b) and 0.13-0.22 mM Gd for the product of Example 6(a).

EXAMPLE 13

13a. Preparation of MnAlPO-5

A solution of 8.2 gm of $Mn(OAc)_2 4H_2O$ in 107.4 gm of water is added to 46.9 gm aluminium isopropoxide in a high shear blender and processed until a thick gel results. To this gel is added a solution containing 46.3 gm of water and 46.2 g of 85% phosphoric acid and 46.9 gm of diethylanolamine. The reaction mixture is mixed until homogeneous and heated for 24 hrs at 200 °C in a sealed vessel. The solids are recovered by filtration, washed with water, and dried in air at ambient temperature. The material is characterized by X-ray powder diffraction and elemental analysis.

13b. Preparation of MnAlPO-11

A solution containing 46.2 gm of 85% phosphoric acid in 92.5 gm of water is added to 21.7 gm of hydrated aluminium oxide. A second solution is prepared by dissolving 19.6 gm of $Mn(OAc)_2 4H_2O$ in 60.1 gm of water followed by 20.2 gm of di-n-propylamine. The solutions are mixed until homogeneous and is heated 24 hrs at 150 °C in a sealed vessel. The solids are recovered by filtration, washed with water, and dried in

air at ambient temperature. The material is characterized by X-ray powder diffraction and elemental analysis.

13c. Calcination of MnAlPO

Samples prepared as in Examples 13(a) or 13(b) are heated to 200 °C in flowing air for 2 hrs followed by a slow heating ca. 10 °C/min to 500 °C and held there for 6 hrs. The sample is cooled and is ready for use. Other crystallographic forms of MnAlPO (i.e. 16, 25, 36, 44, and 47) may be prepared as described in US-A-4567029.

## Claims

**1.** A contrast agent composition for use in a method of diagnostic imaging, said composition comprising a physiologically tolerable carrier or excipient together with a particulate, diagnostically effective metal containing, surface-modified molecular sieve wherein said molecular sieve is surface modified by chemical reaction to constrict the pore openings thereof.

**2.** A composition as claimed in claim 1 for use in a method of magnetic resonance imaging wherein as said diagnostically effective metal said molecular sieve contains paramagnetic metal ions.

**3.** A composition as claimed in claim 1 for use in X-ray imaging wherein as said diagnostically effective metal said molecular sieve contains a metal having an atomic number greater than 37.

**4.** A composition as claimed in any one of claims 1 to 3 wherein said diagnostically effective metal is present in said molecular sieve in a complex with a chelating agent.

**5.** A composition as claimed in claim 4 wherein said complex is cationic.

**6.** A composition as claimed in either of claims 4 and 5 wherein said chelating agent comprises a cationic functional group or a group convertible thereto at physiological pH in an aqueous medium.

**7.** A composition as claimed in any one of claims 1 to 6 comprising at least two different diagnostically effective metals.

**8.** A composition as claimed in any one of claims 1 to 7 wherein said molecular sieve is a zeolite.

**9.** A composition as claimed in any one of claims 1 to 8 comprising a said diagnostically effective metal selected from Gd, Mn and Dy.

**10.** A composition as claimed in any one of claims 1 to 9 wherein said molecular sieve is a MeAPO incorporating as a framework metal Me a divalent diagnostically effective metal having an atomic number of less than 50.

**11.** A composition as claimed in claim 10 wherein said framework metal Me is manganese.

**12.** A composition as claimed in any one of claims 1 to 11 in a form adapted for administration into the gastrointestinal tract.

**13.** The use of a molecular sieve for the manufacture of a contrast agent composition as defined in any one of claims 1 to 12 for use in diagnostic imaging.

**14.** A method of image generation comprising administering to a subject a composition as defined in any one of claims 1 to 12 and generating an image of at least part of said subject.

**15.** A process for the preparation of a molecular sieve as defined in claim 1, said process comprising sequentially or simultaneously: (a) loading a particulate molecular sieve with a diagnostically effective metal; and (b) modifying the surface of the particles of said molecular sieve by reacting with a reagent which serves to constrict the pore openings of said molecular sieve.